# EUROPEAN PATENT APPLICATION

(11) **EP 4 715 833 A1**
(43) Date of publication of application: **25.03.2026**
(21) Application number: 24831634.1
(22) Date of filing: 07.06.2024
(51) Int. Cl.: G16H 20/10

(54) **INFORMATION PROCESSING DEVICE, MEDICAL SYSTEM, AND INFORMATION PROCESSING METHOD**

(30) Priority: 29.06.2023 JP 2023107119
(71) Applicant: Nipro Corporation, Settsu-shi, Osaka 566-8510 (JP)
(72) Inventor: TAKAHASHI, Hidenori, Settsu-shi, Osaka 566-8510 (JP); TAKAHASHI, Koichi, Settsu-shi, Osaka 566-8510 (JP)
(74) Representative: Prüfer & Partner mbB Patentanwälte · Rechtsanwälte
(86) International application number: PCT/JP2024/020926
(87) International publication number: WO 2025/004764

(57) **Abstract**

An information processing device includes: a scanner that reads liquid medicine administration instruction information including a specified value of a setting item for a liquid medicine administration pump, thereby generating data based on the liquid medicine administration instruction information; extraction means that extracts the specified value from the generated data; and transmission means that transmits the extracted specified value to the liquid medicine administration pump.

## Description

### TECHNICAL FIELD

The present disclosure relates to an information processing device, a medical system, and an information processing method.

### BACKGROUND ART

A liquid medicine administration pump such as an infusion pump or a syringe pump has been conventionally used in a hospital and the like. Administration of a liquid medicine to a patient using a liquid medicine administration pump is usually performed by two medical professionals (typically nurses) as follows.

One of the two nurses operates an operation button of the liquid medicine administration pump while looking at liquid medicine administration instruction information such as a medical record of the patient, to make various settings such as a flow rate of the liquid medicine. After setting specified values of setting items such as the flow rate of the liquid medicine, this nurse looks at the medical record again and reads aloud the specified values of the flow rate and the like written on the medical record one by one. When one specified value is read aloud by this nurse, the other nurse checks representation on the liquid medicine administration pump and reads aloud the contents of the monitor representation about this setting. Therefore, when the flow rate and the like are correctly set in the liquid medicine administration pump, the utterance of one of the two nurses is repeated by the other nurse.

Japanese Patent Laying-Open No. 2011-87678 (PTL 1) discloses an infusion pump including a portable setting terminal and a pump main body provided separately from the setting terminal, the setting terminal including an operation unit that sets specified values of a scheduled volume and a flow rate, and a storage unit that stores the command values set by the operation unit. A command value receiver (reader) for performing near field communication with the setting terminal is provided on an upper surface of the pump main body. When a nurse holds the setting terminal over the command value receiver, the specified values of the scheduled volume and the flow rate stored in the storage unit of the setting terminal are loaded into the pump main body. The pump main body performs a pump operation in accordance with the loaded specified values of the scheduled volume and the flow rate.

### CITATION LIST

### PATENT LITERATURE

PTL 1: Japanese Patent Laying-Open No. 2011-87678

### SUMMARY OF INVENTION

### TECHNICAL PROBLEM

In PTL 1, when the nurse misreads a medical record, an incorrect setting is made in the liquid medicine administration pump. Furthermore, in PTL 1, even when the nurse reads the medical record correctly, an incorrect setting is made in the liquid medicine administration pump if the nurse performs an incorrect input to the portable setting terminal. In either case, if one of the two nurses notices his/her mistake in setting through double-checking with the other nurse, this nurse can reset the setting. However, the occurrence of a human input error when making a setting in the liquid medicine administration pump should be prevented.

An object of the present disclosure is to provide an information processing device, a medical system, and an information processing method, which make it possible to appropriately set a specified value written on liquid medicine administration instruction information for a setting item in a liquid medicine administration pump.

### SOLUTION TO PROBLEM

According to an aspect of the present disclosure, an information processing device includes: a scanner that reads liquid medicine administration instruction information including a specified value of a setting item for a liquid medicine administration pump, thereby generating first data based on the liquid medicine administration instruction information; extraction means that extracts the specified value from the generated first data; and transmission means that transmits the extracted specified value to the liquid medicine administration pump.

According to another aspect of the present disclosure, a medical system includes: the above-described information processing device; and the liquid medicine administration pump.

According to still another aspect of the present disclosure, an information processing method includes: reading, with a sensor, liquid medicine administration instruction information including a specified value of a setting item for a liquid medicine administration pump, thereby generating data; extracting the specified value from the generated data; and transmitting the extracted specified value to the liquid medicine administration pump.

### ADVANTAGEOUS EFFECTS OF INVENTION

According to the above-described configuration, it is possible to appropriately set a specified value written on liquid medicine administration instruction information for a setting item in a liquid medicine administration pump.

### BRIEF DESCRIPTION OF DRAWINGS

Fig. 1 is a diagram showing a configuration of a medical system.
Fig. 2 is a diagram showing details of data to be transmitted to a liquid medicine pump.
Fig. 3 is a diagram showing a state in which a nurse is operating a tablet terminal.
Fig. 4 is a diagram showing an example of an electronic medical record displayed on a display of a computer.
Fig. 5 is a diagram for illustrating data processing performed by the tablet terminal.
Fig. 6 is a diagram showing a user interface of the tablet terminal.
Fig. 7 is a diagram showing a user interface displayed on the tablet terminal after a scanning process ends.
Fig. 8 is a block diagram for illustrating configurations of the tablet terminal and an infusion pump.
Fig. 9 is a sequence diagram showing a flow of a process performed in the medical system.
Fig. 10 is a diagram for illustrating a configuration for scanning a pharmaceutical label with the tablet terminal.
Fig. 11 is a diagram for illustrating a configuration for scanning an injection prescription with the tablet terminal.
Fig. 12 is a diagram for illustrating a modification of the tablet terminal.
Fig. 13 is a diagram showing a modification of the medical system.

### DESCRIPTION OF EMBODIMENTS

Hereinafter, an embodiment of the present disclosure will be described with reference to the drawings. In the description below, the same members will be denoted by the same reference characters. Since their names and functions are also the same, detailed description thereof will not be repeated.

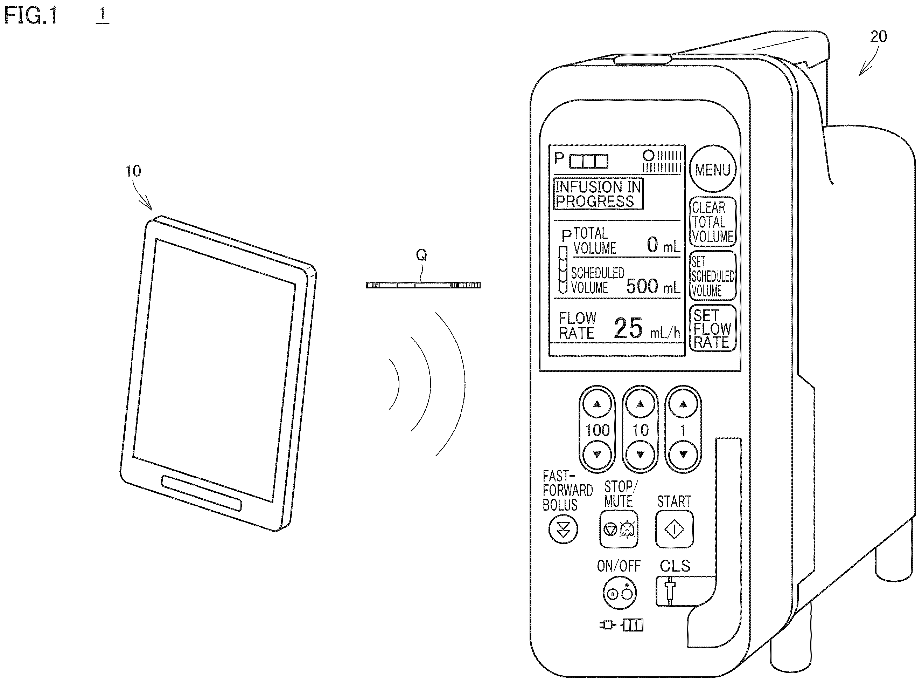

Fig. 1 is a diagram showing a configuration of a medical system. As shown in Fig. 1, a medical system 1 includes a tablet terminal 10, which is an example of an information processing device, and an infusion pump 20, which is an example of a liquid medicine administration pump. The information processing device is not limited to tablet terminal 10 and may be a portable device such as a smartphone or a laptop-type (notebook-type) personal computer. The liquid medicine administration pump is not limited to the infusion pump and may be another pump such as a syringe pump.

Tablet terminal 10 communicates with infusion pump 20. Tablet terminal 10 wirelessly communicates with the infusion pump. Tablet terminal 10 performs near field communication (NFC) with infusion pump 20. In the present example, tablet terminal 10 communicates with infusion pump 20, using Felica (registered trademark) (NFC Type-F).

Tablet terminal 10 has a reader/writer for Felica built thereinto. The reader/writer has a control board and an antenna. Infusion pump 20 has Felica Link mounted therein. Infusion pump 20 has a non-contact-type integrated circuit card for Felica (FeliCa Plug) built thereinto. The non-contact-type integrated circuit card has an integrated circuit chip and an antenna.

In the present example, by bringing tablet terminal 10 operating as a reader/writer closer to infusion pump 20 operating as a tag, near field communication is performed between tablet terminal 10 and infusion pump 20. The reason why tablet terminal 10, not infusion pump 20, has the reader/writer built thereinto is that tablet terminal 10 also performs near field communication with other pumps not shown in the figure, similarly to infusion pump 20.

Infusion pump 20 includes a plurality of operation buttons and a display. Each of the operation buttons is operated by a doctor or a nurse. For example, various setting items such as a flow rate, a scheduled volume and a total volume of a liquid medicine can be input through the operation buttons. Furthermore, the operation buttons can instruct infusion pump 20 to start and end the operation.

Tablet terminal 10 transmits data Q to infusion pump 20 through the above-described near field communication. Data Q is a lump of data. Data Q is a frame (packet). Data Q may be data referred to as a segment, a datagram or a message. Infusion pump 20 receives data Q.

As will be described in detail below, when infusion pump 20 receives data Q, infusion pump 20 sets various setting items based on data Q. For example, infusion pump 20 automatically sets the flow rate, the scheduled volume, the total volume and the like of the liquid medicine. In this way, in medical system 1, input of various setting items to infusion pump 20 is automated.

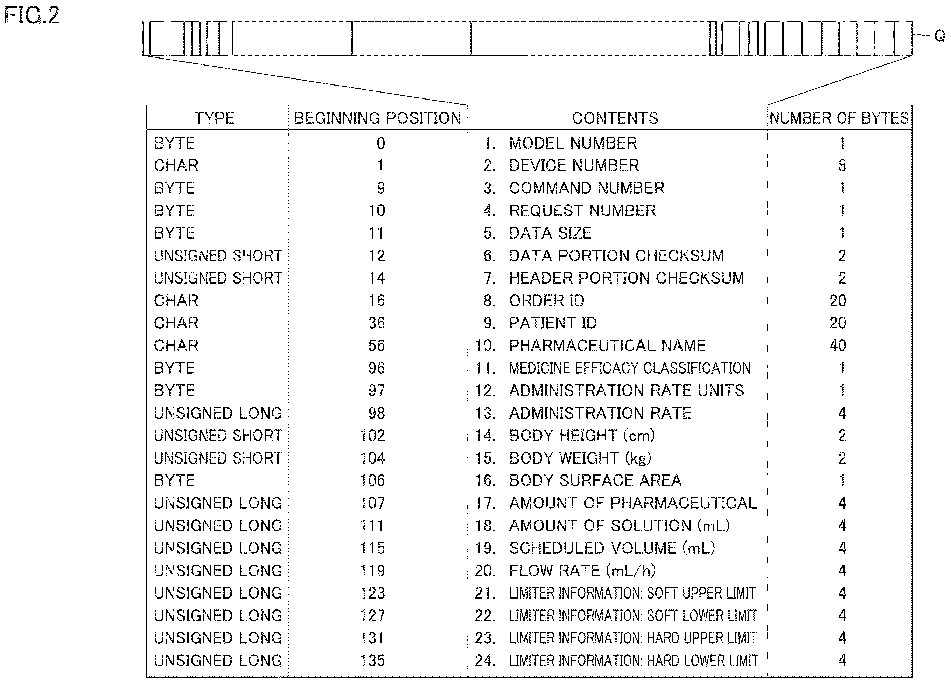

Fig. 2 is a diagram showing details of data Q. A plurality of pieces of information is written in data Q. Data Q includes a header portion and a data portion. The header portion has seven pieces of information. The data portion has seventeen pieces of information.

Specifically, the pieces of information about "model number", "device number", "command number", "request number", "data size", "data portion checksum", and "header portion checksum" are written in the header portion in this order and from a corresponding beginning position.

The pieces of information about "order ID", "patient ID", "pharmaceutical name", "medicine efficacy classification", "administration rate unit", "administration rate", "body height (cm)", "body weight (kg)", "body surface area", "amount of pharmaceutical", "amount of solution (mL)", "scheduled volume (mL)", "flow rate (mL/h)", "limiter information: soft upper limit", "limiter information: soft lower limit", "limiter information: hard upper limit", and "limiter information: hard lower limit" are written in the data portion in this order and from a corresponding beginning position.

The hard lower limit, the soft lower limit, the soft upper limit, and the hard upper limit become higher in numerical value in this order. "Hard lower limit (upper limit)" indicates a condition that should be strictly complied with, and "soft lower limit (upper limit)" indicates a condition that is looser than "hard lower limit (upper limit)".

As described above, the twenty-four pieces of information can be stored in data Q. All of the twenty-four pieces of information do not need to be written in data Q at the time of transmission from tablet terminal 10 to infusion pump 20. In addition, the type of the information stored in data Q is not limited to the above. For example, data Q may include information about a scheduled administration time of the liquid medicine.

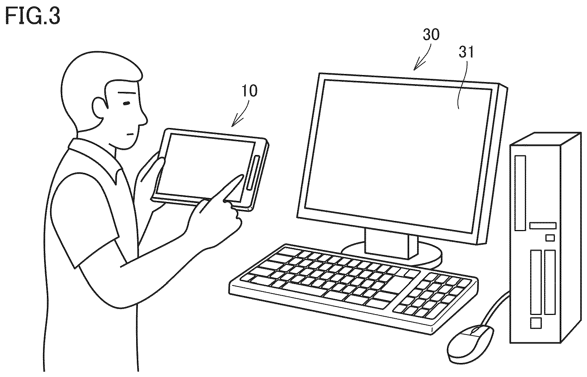

Fig. 3 is a diagram showing a state in which a nurse is operating tablet terminal 10. As shown in Fig. 3, the nurse scans, with tablet terminal 10, an electronic medical record (an example of liquid medicine administration instruction information) displayed on a display 31 of a computer 30 constituting medical system 1. The nurse scans the electronic medical record with a scanner of tablet terminal 10. In the present example, the scanner has the optical character recognition/reader (OCR) function.

Tablet terminal 10 generates image data by image pickup with a sensor (camera) of the scanner. Tablet terminal 10 recognizes a text in the image data with a data processing unit (OCR processing unit) of the scanner. Thus, tablet terminal 10 converts the text in the image data into character data.

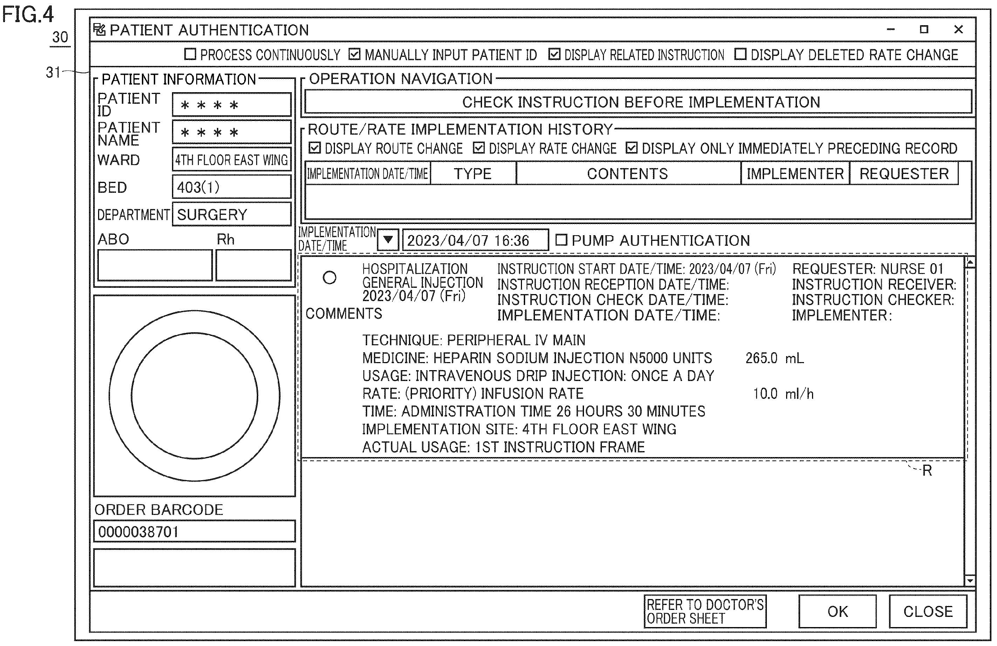

Fig. 4 is a diagram showing an example of the electronic medical record displayed on display 31 of computer 30. The nurse scans, with tablet terminal 10, a portion of a region R in the electronic medical record. The contents of the electronic medical record shown in Fig. 4 are merely an example and the present disclosure is not limited thereto. The terminal on which the electronic medical record is displayed is not limited to a desktop-type computer shown in Fig. 4 and may be a mobile terminal.

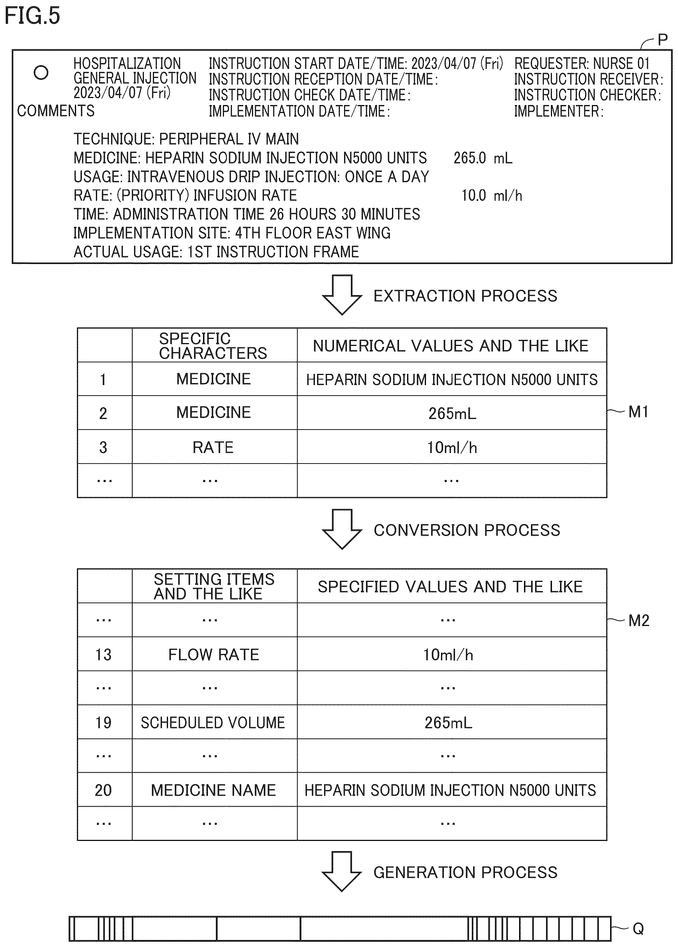

Fig. 5 is a diagram for illustrating data processing performed by tablet terminal 10. As shown in Fig. 5, tablet terminal 10 scans region R shown in Fig. 4, thereby acquiring data P in text format. The data contents of data P shown in Fig. 5 are merely an example and the present disclosure is not limited thereto.

Tablet terminal 10 extracts, from data P, a specific character that is specified in advance, and information such as a numerical value related to the specific character. For example, since there is a character string "medicine: heparin sodium injection N5000 units 265.0 mL" on the sixth line from the bottom of data P, "heparin sodium injection N5000 units" and "265.0 mL" are associated with "medicine".

Specifically, tablet terminal 10 learns a form of the electronic medical record in advance. Tablet terminal 10 learns what information is written in which region of the electronic medical record. Tablet terminal 10 recognizes the order of the information written on the electronic medical record including region R in advance. Specifically, tablet terminal 10 stores template data indicating a template of the electronic medical record shown in Fig. 4.

Tablet terminal 10 refers to the template data and performs the above-described extraction process, thereby generating data M1. Specifically, tablet terminal 10 compares the characters of the items, the icons of the items, arrangement (order) of the items, the units and the like in data P with the template data, thereby generating data M1.

Tablet terminal 10 generates data M2 based on data M1. Tablet terminal 10 executes a prescribed program to convert data M1 into a form of data M2. Data M2 has an item "setting items and the like" and an item "specified values and the like". The item "contents" in data Q shown in Fig. 2 corresponds to the item "setting items and the like" and the item "specified values and the like" in data M2.

In data M1, "medicine" and "heparin sodium injection N5000 units" are associated with each other. "Medicine" and "265.0 mL" are associated with each other. "Rate" and "10 ml/h" are associated with each other.

Therefore, tablet terminal 10 associates "heparin sodium injection N5000 units" with the twentieth item "medicine name" in data M2. Tablet terminal 10 associates "265.0 mL" with the nineteenth item "scheduled volume" in data M2. Tablet terminal 10 associates "10 ml/h" with the thirteenth item "flow rate" in data M2. "Twentieth", "nineteenth" and "thirteenth" correspond to the numbers of the contents in data Q shown in Fig. 2.

In data M1, the two pieces of information are associated with the specific character "medicine". In this case, tablet terminal 10 determines which information is associated with which item in data M2. For example, tablet terminal 10 associates "heparin sodium injection N5000 units" with "medicine name" and "265.0 mL" with "scheduled volume" as described above, based on the numerical value, the unit, the length of the character string, character matching, the positional information in data P, and the like.

In data M1, "10 ml/h" is associated with the specific character "rate". Tablet terminal 10 reads "rate" as "flow rate" and associates "10 ml/h" with the thirteenth item "flow rate" in data M2.

Tablet terminal 10 generates data Q based on data M2. Specifically, a processor of tablet terminal 10 writes each of the extracted specified values to a communication format having the same frame structure as that of data Q, thereby generating data Q.

When the nurse brings tablet terminal 10 closer to infusion pump 20 and performs a transmission operation on tablet terminal 10, tablet terminal 10 transmits data Q to infusion pump 20.

When infusion pump 20 receives data Q, infusion pump 20 sets various setting items based on data Q as described above. For example, infusion pump 20 automatically sets "scheduled volume" to "265.0 mL". The infusion pump automatically sets "flow rate" to "10 ml/h". In the present example, "scheduled volume" and "flow rate" are examples of the setting item, and "265.0 mL" and "10 ml/h" are examples of the specified value.

Hereinafter, for the sake of convenience in description, the data (data in text format) generated from the liquid medicine administration instruction information (in the present example, the electronic medical record) will be referred to as data P, regardless of the contents of the liquid medicine administration instruction information. Furthermore, the data having the above-described frame structure (see Fig. 2), which is generated from data P, will be referred to as data Q, regardless of the contents of the liquid medicine administration instruction information.

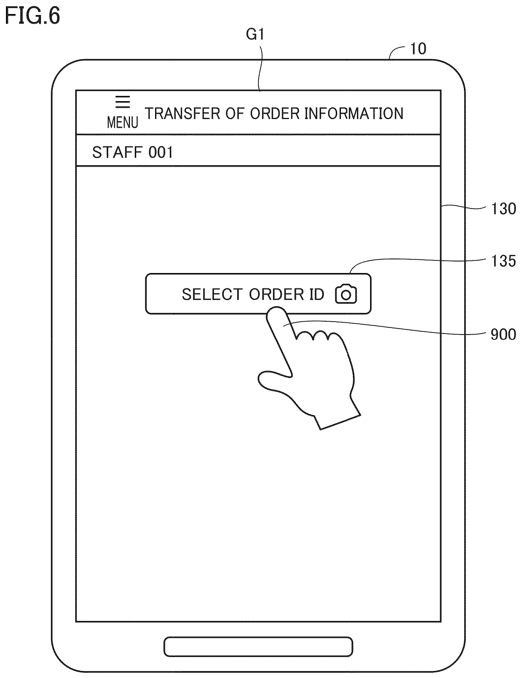

Fig. 6 is a diagram showing a user interface (screen) of tablet terminal 10. Referring to Fig. 6, tablet terminal 10 includes a touch screen 130 having a display and a touch panel. Tablet terminal 10 stores an application program.

When the nurse operates tablet terminal 10 to cause tablet terminal 10 (specifically, the processor) to execute the above-described application program, tablet terminal 10 displays an initial screen G1. Initial screen G1 includes a selectable object image 135 indicating "select order ID".

When the nurse selects object image 135 with his/her finger 900, tablet terminal 10 performs the above-described scanning process. As a result, tablet terminal 10 sequentially generates above-described data M1, M2 and Q.

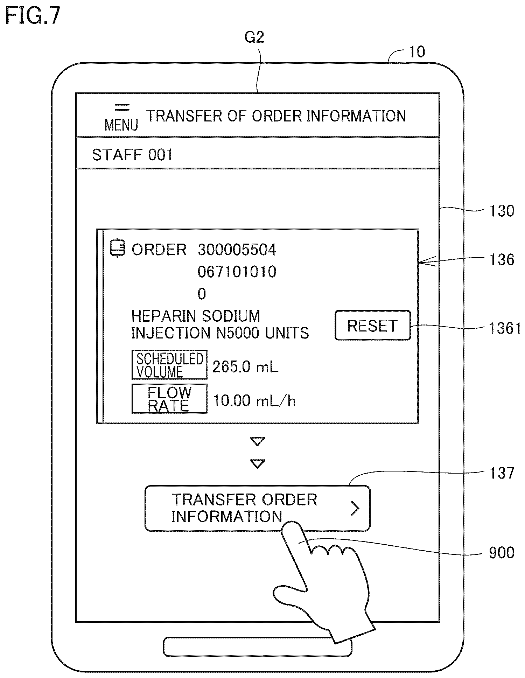

Fig. 7 is a diagram showing a user interface displayed on tablet terminal 10 after the scanning process ends. As shown in Fig. 7, the tablet terminal displays a screen G2 on touch screen 130. Screen G2 includes an object image 136 indicating setting information, and a selectable object image 137 indicating "transfer order information". The setting information includes the specified values of the setting items.

Object image 136 includes information based on data M2 (or data Q). In the present example, "heparin sodium injection N5000 units" is displayed as "medicine name" in object image 136. "265.0 mL" and "10 ml/h" are displayed as "scheduled volume" and "flow rate" in object image 136, respectively. The hidden information based on data M2 can be displayed through scrolling by the nurse.

Object image 136 includes a selectable object image 1361 indicating "reset". When the nurse selects object image 1361, "medicine name", "scheduled volume" and "flow rate" become changeable. Specifically, the tablet terminal switches the screen from screen G2 to another screen for changing and accepts a change instruction (user input) on the switched screen. By selecting object image 1361, a numerical value (of a specified value) of a setting item that has not yet been acquired can also be added.

When the nurse selects object image 137 with his/her finger 900, tablet terminal 10 transmits data Q to infusion pump 20. As described above, tablet terminal 10 can transfer the order information about the liquid medicine written on the electronic medical record to the infusion pump as data Q. Before transmission, the nurse can check, on tablet terminal 10, data Q to be transmitted to infusion pump 20.

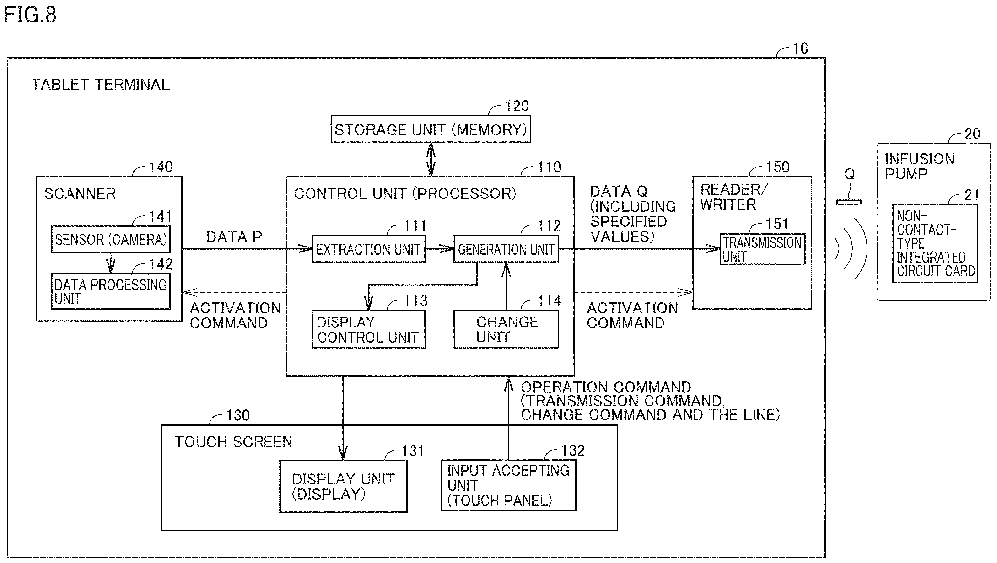

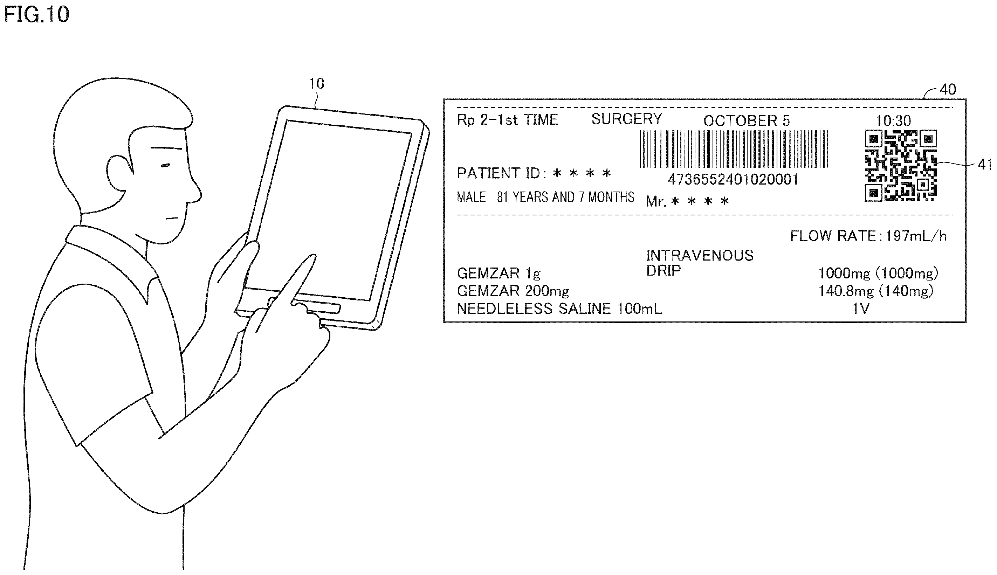

Fig. 8 is a block diagram for illustrating configurations of tablet terminal 10 and infusion pump 20. As shown in Fig. 10, infusion pump 20 includes a non-contact-type integrated circuit card 21. Tablet terminal 10 includes a control unit 110, a storage unit 120, touch screen 130, a scanner 140, and a reader/writer 150.

Control unit 110 includes an extraction unit 111, a generation unit 112, a display control unit 113, and a change unit 114. Touch screen 130 includes a display unit 131 and an input accepting unit 132. In the present example, display unit 131 corresponds to the above-described display. Input accepting unit 132 corresponds to the touch panel. Scanner 140 includes a sensor 141 and a data processing unit 142. In the present example, sensor 141 is a camera. Reader/writer 150 includes a transmission unit 151.

Control unit 110 is a functional block implemented by execution of an operating system and various application programs stored in storage unit 120 corresponding to the memory by the processor. The above-described communication format is prestored in storage unit 120.

Scanner 140 reads the liquid medicine administration instruction information including the specified values of the setting items for infusion pump 20, thereby generating data based on the liquid medicine administration instruction information. In the present example, scanner 140 reads the electronic medical record. When scanner 140 reads the electronic medical record shown in Fig. 4, scanner 140 generates data P shown in Fig. 5.

Specifically, sensor 141 of scanner 140 picks up an image of the electronic medical record. Data processing unit 142 has the OCR function. Data processing unit 142 generates data P (text data) from image data obtained by image pickup with sensor 141.

Control unit 110 controls the overall operation of tablet terminal 10. Control unit 110 communicates with storage unit 120, touch screen 130, scanner 140, and reader/writer 150. Control unit 110 transmits an activation command to scanner 140, reader/writer 150 and the like.

Extraction unit 111 extracts each of the specified values of the plurality of setting items from data P transmitted from the scanner. In the example shown in Fig. 5, extraction unit 111 generates data M1 and data M2 from data P.

Generation unit 112 writes each of the specified values extracted by extraction unit 111 to the above-described communication format stored in storage unit 120, thereby generating data Q for communicating with infusion pump 20. In the example shown in Fig. 5, generation unit 112 generates data Q from data M2. Generation unit 112 transmits generated data Q to reader/writer 150.

Reader/writer 150 transmits data Q to infusion pump 20. Specifically, transmission unit 151 transmits data Q to infusion pump 20 through near field communication. Infusion pump 20 receives data Q with non-contact-type integrated circuit card 21.

Display control unit 113 causes display unit 131 to display various screens. For example, display control unit 113 causes display unit 131 to display initial screen G1 shown in Fig. 6. Display control unit 113 causes display unit 131 to display screen G2 based on data M2 generated by generation unit 112 as shown in Fig. 7.

Input accepting unit 132 accepts various user inputs (touch operations). For example, input accepting unit 132 accepts an input to change each of the extracted specified values. In this case, change unit 114 changes the specified value to a value based on the input. Such a change process is performed after object image 1361 shown in Fig. 7 is selected. In this case, changed data Q is transmitted to infusion pump 20. Therefore, before tablet terminal 10 transmits data Q to infusion pump 20, a user can change the information such as the numerical value as needed.

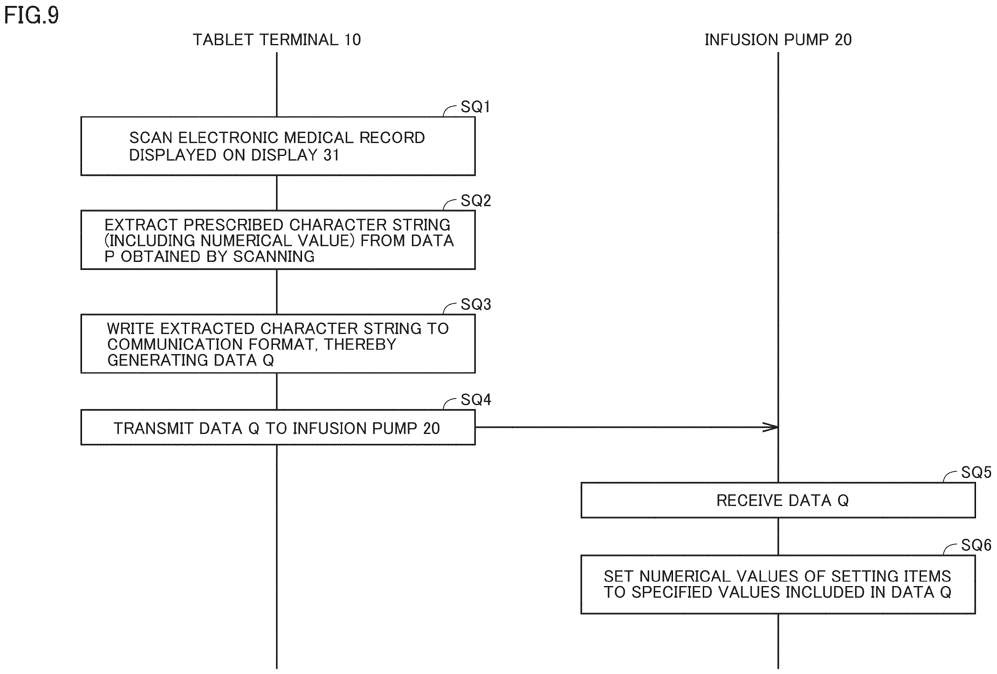

Fig. 9 is a sequence diagram showing a flow of a process performed in medical system 1. In sequence SQ1, tablet terminal 10 scans an electronic medical record displayed on display 31 of computer 30. In sequence SQ2, tablet terminal 10 (specifically, extraction unit 111) extracts a prescribed character string (including a numerical value) from data P obtained by scanning. In sequence SQ3, tablet terminal 10 (specifically, generation unit 112) writes the extracted character string to a communication format, thereby generating data Q. In sequence SQ4, tablet terminal 10 (specifically, transmission unit 151) transmits data Q to infusion pump 20 through near field communication.

In sequence SQ5, infusion pump 20 receives data Q. In sequence SQ6, infusion pump 20 sets numerical values of a plurality of setting items to the numerical values included in data Q.

### <Brief Summary>

Referring to Fig. 5 and the like, a part of the configuration of tablet terminal 10 and the like are briefly summarized as follows.
(1) Tablet terminal 10 includes scanner 140 that reads an electronic medical record including a specified value of a setting item for infusion pump 20, thereby generating data P based on the electronic medical record. Tablet terminal 10 further includes extraction unit 111 that extracts the specified value from generated data P. Tablet terminal 10 further includes transmission unit 151 that transmits the extracted specified value to infusion pump 20. Examples of the specified value include a flow rate of a liquid medicine, a scheduled volume of the liquid medicine, and a scheduled administration time of the liquid medicine.

According to such a configuration, an incorrect setting caused by misreading of the electronic medical record by the nurse can be prevented from being made in infusion pump 20. Furthermore, since the nurse does not need to input any specified value to infusion pump 20, an incorrect input can be prevented. Therefore, it is possible to prevent specified values different from the specified values written on the electronic medical record from being input to infusion pump 20. Specifically, it is possible to prevent the nurse from inputting the wrong number of digits in numerical value, or the nurse from inputting a numerical value to a different setting item.

Therefore, according to medical system 1, the specified value written on the electronic medical record, which is an example of the liquid medicine administration instruction information, can be appropriately set in infusion pump 20, which is an example of the liquid medicine administration pump. Therefore, one nurse can perform a series of setting work in infusion pump 20. Specifically, since double-checking is unnecessary, one nurse can perform the series of setting work.

(2) Specifically, the electronic medical record includes each of the specified values of the plurality of setting items. Extraction unit 111 extracts each of the specified values from data P. Tablet terminal 10 further includes generation unit 112 that generates data Q for communicating with infusion pump 20, by writing each of the specified values extracted by extraction unit 111 to a predetermined communication format. The transmission unit transmits data Q to infusion pump 20. According to such a configuration, data Q makes it possible to set each of the specified values of the plurality of setting items in infusion pump 20.

### <Modifications>

### (First Modification)

In the example described above, tablet terminal 10 scans the electronic medical record displayed on display 31 of computer 30. However, the present disclosure is not limited to the above-described example.

Fig. 10 is a diagram for illustrating a configuration for scanning a pharmaceutical label with tablet terminal 10. As shown in Fig. 10, liquid medicine administration instruction information is written on a pharmaceutical label 40. Specifically, various pieces of information such as a patient ID, a patient name, a name of a liquid medicine, an amount of administration of the liquid medicine, and a flow rate (administration rate) of the liquid medicine are written on pharmaceutical label 40. As described above, the liquid medicine administration instruction information is written on pharmaceutical label 40.

The nurse scans pharmaceutical label 40 with tablet terminal 10. As a result of scanning of pharmaceutical label 40, tablet terminal 10 acquires data P in text format, as is the case with the electronic medical record. Thereafter, tablet terminal 10 performs the above-described extraction process and generation process in this order (see Fig. 5), thereby generating data Q. In this case as well, tablet terminal 10 transmits data Q to infusion pump 20. As described above, infusion pump 20 sets various setting items based on data Q.

In the present example, a two-dimensional code 41 having these pieces of information stored thereon is written on pharmaceutical label 40. Thus, the liquid medicine administration instruction information is provided as two-dimensional code 41. Two-dimensional code 41 is typically a QR code (registered trademark). When an application program for reading the two-dimensional code is pre-installed on tablet terminal 10 (scanner), tablet terminal 10 may read two-dimensional code 41.

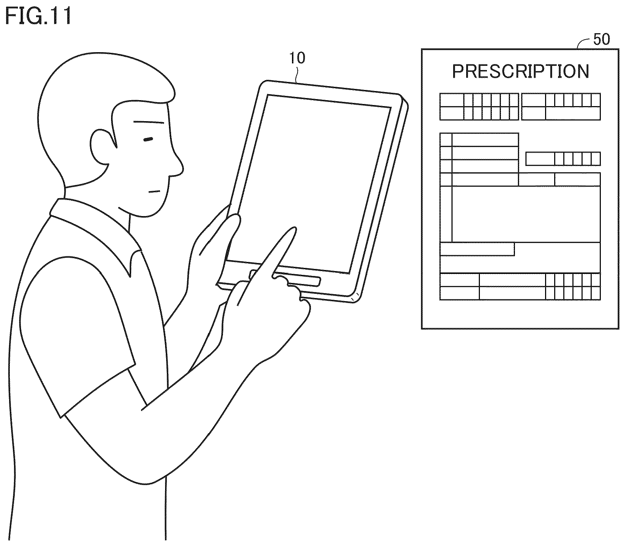

Fig. 11 is a diagram for illustrating a configuration for scanning an injection prescription with tablet terminal 10. Liquid medicine administration instruction information is written on an injection prescription 50 shown in Fig. 11. Specifically, a patient name, a prescription and the like are written on the injection prescription. A name of a liquid medicine, an amount of administration, an administration rate and the like are written in the prescription section.

The nurse scans injection prescription 50 with tablet terminal 10. As a result of scanning of injection prescription 50, tablet terminal 10 acquires data P in text format, as is the case with the electronic medical record and pharmaceutical label 40. Thereafter, tablet terminal 10 performs the above-described extraction process and generation process in this order (see Fig. 5), thereby generating data Q. In this case as well, tablet terminal 10 transmits data Q to infusion pump 20. Infusion pump 20 sets various setting items based on data Q as described above.

As described above, the nurse may acquire the liquid medicine administration instruction information from not only the electronic medical record but also the pharmaceutical label and the injection prescription. Alternatively, the nurse may acquire the liquid medicine administration instruction information by scanning a paper-based medical record (e.g., a printout of the electronic medical record or a handwritten medical record). As long as the liquid medicine administration instruction information is displayed or printed, a medium to be scanned is not particularly limited.

### (Second Modification)

A format of the electronic medical record varies depending on a vendor that provides an electronic medical record system or a hospital that uses an electronic medical record system. The pharmaceutical label and the injection prescription also vary. Therefore, in order to allow tablet terminal 10 to transmit the specified values of the setting items to infusion pump 20, extraction unit 111 needs to extract the specified values from data P based on the format of the electronic medical record, the pharmaceutical label or the injection prescription. In addition, the frame structure used for communication with infusion pump 20 varies, unless it is standardized. Therefore, the frame structure used for communication needs to be prepared for each manufacturer or model. A tablet terminal that can deal with such a situation will be described below.

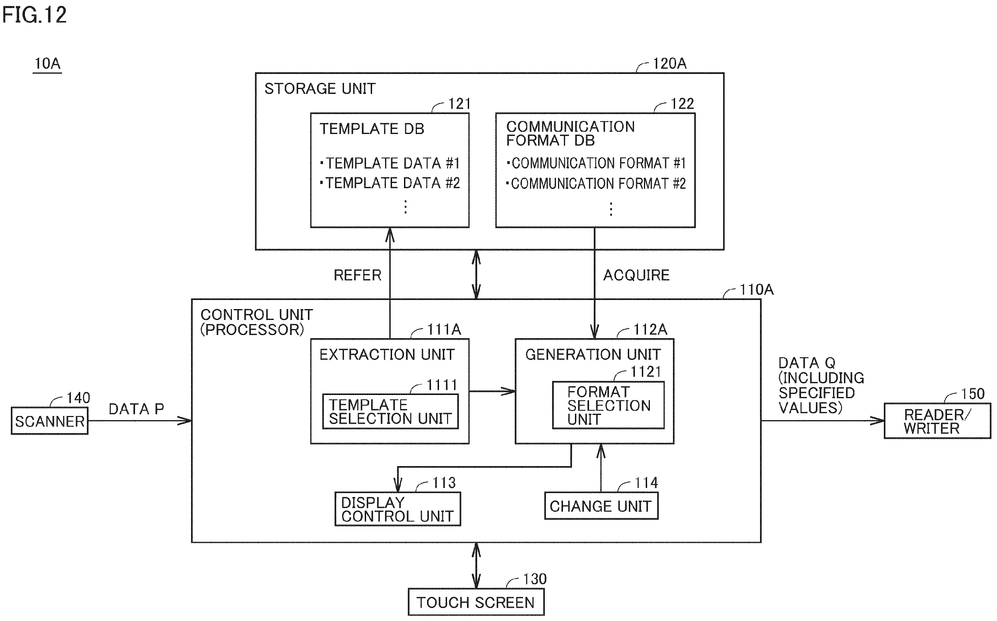

Fig. 12 is a diagram for illustrating a modification of tablet terminal 10. As shown in Fig. 12, a tablet terminal 10A as a modification includes a control unit 110A, a storage unit 120A, touch screen 130, scanner 140, and reader/writer 150. Tablet terminal 10A is different from tablet terminal 10 including control unit 110 and storage unit 120, in that tablet terminal 10A includes control unit 110A and storage unit 120A.

Control unit 110A includes an extraction unit 111A, a generation unit 112A, display control unit 113, and change unit 114. Control unit 110A is different from control unit 110 including extraction unit 111 and generation unit 112, in that control unit 110A includes extraction unit 111A and generation unit 112A. Extraction unit 111A has a template selection unit 1111. Generation unit 112A has a format selection unit 1121.

Storage unit 120A stores a template data database (DB) 121 and a communication format DB 122. Template data DB 121 has a plurality of templates #1, #2, .... Communication format DB 122 has a plurality of communication formats #1, #2, ...

Template selection unit 1111 refers to data P and selects template data corresponding to data P from template data DB 121. Without being limited to the above, template selection unit 1111 may select one piece of template data from a plurality of pieces of template data based on image data acquired by sensor 141. Template selection unit 1111 performs pattern matching between data P or the above-described image data and the plurality of pieces of template data.

Extraction unit 111A refers to the selected template data and performs an extraction process for extracting a specified value. According to such a configuration, before the extraction process, extraction unit 111A can recognize which information is written on which part of the liquid medicine administration instruction information, based on the template data. Therefore, a relationship between the setting item and the specified value can be appropriately understood. Therefore, extraction unit 111A can accurately generate data M2.

Generation unit 112A generates data Q from data M2. At this time, format selection unit 1121 selects a communication format corresponding to a model of infusion pump 20 from a plurality of communication formats. The information about the model of infusion pump 20 and the like may be acquired by tablet terminal 10A through preliminary communication and the like. Using the selected communication format, generation unit 112A generates data Q to be transmitted to infusion pump 20.

Selection of the template data is not limited to the above-described automatic selection by tablet terminal 10. The nurse or the like may select the template data corresponding to the liquid medicine administration instruction information to be scanned from the plurality of pieces of template data in advance.

### (Third Modification)

In the example described above, medical system 1 includes only tablet terminal 10 as the information processing device. However, the present disclosure is not limited to the above-described example. The process by tablet terminal 10 may be distributed to the tablet terminal and a server device. The information processing device may include the tablet terminal and the server device. Such a configuration will be described below.

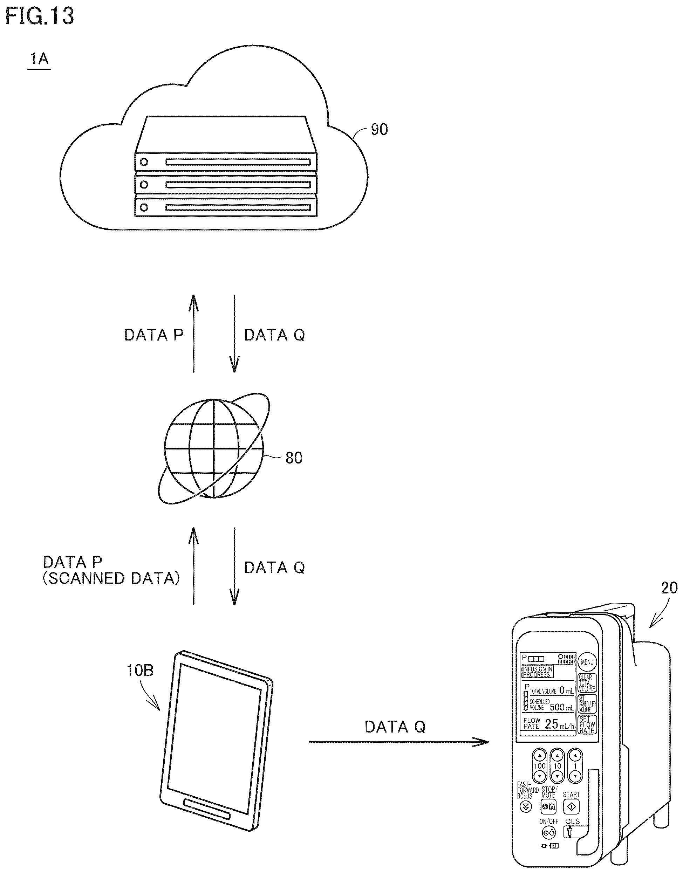

Fig. 13 is a diagram showing a modification of medical system 1. As shown in Fig. 13, a medical system 1A as a modification includes a tablet terminal 10B, infusion pump 20 and a server device 90. Tablet terminal 10B and server device 90 are connected to each other through a network such as the Internet.

Tablet terminal 10B scans, with scanner 140, liquid medicine administration instruction information such as an electronic medical record, thereby generating data P (see Fig. 5), similarly to tablet terminal 10. Tablet terminal 10B transmits data P to server device 90. Server device 90 generates data M1 and data M2 based on data P. Server device 90 further generates data Q based on data M2. Server device 90 transmits generated data Q to tablet terminal 10B serving as a sender of data P. Tablet terminal 10B transmits data Q received from server device 90 to infusion pump 20 through near field communication using reader/writer 150.

As described above, a series of process performed by tablet terminal 10 is distributed to tablet terminal 10B and server device 90, which makes it possible to reduce a load on the tablet terminal 10B side. Specifically, server device 90 performs the above-described processes by extraction unit 111 and generation unit 112.

Since server device 90 is used, a heavy-load processing method can also be used to generate data Q from data P. For example, a trained model can be prestored in server device 90 and data Q can be generated from data P using the trained model. Specifically, server device 90 can generate data Q from data P using a trained model that functions as above-described extraction unit 111 and generation unit 112. As described above, by inputting data P to the trained model, data Q can be obtained as output data.

More specifically, server device 90 learns various pieces of liquid medicine administration instruction information having different formats (forms) (typically, liquid medicine administration instruction information customized for each hospital) in advance. Specifically, training is performed such that a piece of template data corresponding to data P can be specified from a plurality of pieces of template data. Using a trained model obtained by such a training, server device 90 can refer to the template data corresponding to data P and perform the above-described extraction process. Therefore, according to server device 90, appropriate data Q can be generated regardless of the format of the liquid medicine administration instruction information.

Server device 90 may specify the template data based on image data (data before being converted to a text format) obtained as a result of scanning of the liquid medicine administration instruction information, instead of data P. In this case, tablet terminal 10B may transmit only the image data to server device 90.

### (Fourth Modification)

In medical system 1 that does not include server device 90, tablet terminal 10 may have the above-described trained model.

According to medical systems 1 and 1A described above, the liquid medicine administration instruction information such as the electronic medical record is read, whereby data Q for infusion pump 20 is generated. Therefore, the order information about the liquid medicine written on the above-described liquid medicine administration instruction information can be transferred without collaborating with a vendor that provides the electronic medical record or the like. Specifically, notification about the information (such as the flow rate of the liquid medicine) included in the order information can be provided to infusion pump 20.

### <Additional Notes>

### [Item 1]

An information processing device comprising:
a scanner that reads liquid medicine administration instruction information including a specified value of a setting item for a liquid medicine administration pump, thereby generating first data based on the liquid medicine administration instruction information;
extraction means that extracts the specified value from the generated first data; and
transmission means that transmits the extracted specified value to the liquid medicine administration pump.

### [Item 2]

The information processing device according to Item 1, wherein
the liquid medicine administration instruction information includes a plurality of the specified values corresponding to a plurality of the setting items, respectively,
the extraction means extracts each of the specified values from the first data,
the information processing device further includes generation means that generates second data for communicating with the liquid medicine administration pump, by writing each of the extracted specified values to a predetermined communication format, and
the transmission means transmits the second data to the liquid medicine administration pump.

### [Item 3]

The information processing device according to Item 1 or 2, wherein
the liquid medicine administration instruction information includes at least one of a flow rate of a liquid medicine as the specified value, a scheduled volume of the liquid medicine as the specified value, and a scheduled administration time of the liquid medicine as the specified value.

### [Item 4]

The information processing device according to any one of Items 1 to 3, wherein
the scanner reads a pharmaceutical label on which the liquid medicine administration instruction information is written, an injection prescription on which the liquid medicine administration instruction information is written, a display screen of an electronic medical record on which the liquid medicine administration instruction information is written, or a paper-based medical record on which the liquid medicine administration instruction information is written, thereby generating the first data.

### [Item 5]

The information processing device according to Item 2, wherein
the information processing device includes a terminal device and a server device capable of communicating with the terminal device,
the server device includes the extraction means and the generation means, and
the terminal device includes the scanner and the transmission means.

### [Item 6]

The information processing device according to Item 5, wherein
the server device includes a trained model that functions as the extraction means and the generation means, and generates the second data from the first data using the trained model.

### [Item 7]

The information processing device according to any one of Items 1 to 6, wherein
the liquid medicine administration instruction information is provided as a two-dimensional code, and
the scanner includes an application program that reads the two-dimensional code.

### [Item 8]

The information processing device according to any one of Items 1 to 7, wherein
the transmission means transmits the specified value to the liquid medicine administration pump through near field communication.

### [Item 9]

The information processing device according to Item 8, wherein
the liquid medicine administration pump has a non-contact-type integrated circuit card,
the information processing device is a portable device including a reader/writer that functions as at least the transmission means, and
when the information processing device is brought closer to the liquid medicine administration pump, the information processing device transmits the specified value to the liquid medicine administration pump using the reader/writer.

### [Item 10]

The information processing device according to any one of Items 1 to 9, further comprising:
input accepting means that accepts an input to change the extracted specified value; and
change means that changes the specified value to a value based on the input, wherein
the transmission means transmits the changed specified value to the liquid medicine administration pump.

### [Item 11]

A medical system comprising:
the information processing device according to any one of Items 1 to 10; and
the liquid medicine administration pump.

It should be understood that the embodiment disclosed herein is illustrative and non-restrictive in every respect. The scope of the present disclosure is defined by the terms of the claims and is intended to include any modifications within the scope and meaning equivalent to the terms of the claims.

### REFERENCE SIGNS LIST

1, 1A medical system; 10, 10A, 10B tablet terminal; 20 infusion pump; 21 non-contact-type integrated circuit card; 30 computer; 31 display; 40 pharmaceutical label; 41 two-dimensional code; 50 injection prescription; 90 server device; 110, 110A control unit; 111, 111A extraction unit; 112, 112A generation unit; 113 display control unit; 114 change unit; 120, 120A storage unit; 130 touch screen; 131 display unit; 132 input accepting unit; 135, 136, 137, 1361 object image; 140 scanner; 141 sensor; 142 data processing unit; 150 reader/writer; 151 transmission unit; 900 finger; 1111 template selection unit; 1121 format selection unit; G1 initial screen; G2 screen; M1, M2, P, Q data; R region.

## Claims

1. An information processing device comprising:
a scanner that reads liquid medicine administration instruction information including a specified value of a setting item for a liquid medicine administration pump, thereby generating first data based on the liquid medicine administration instruction information;
extraction means that extracts the specified value from the generated first data; and
transmission means that transmits the extracted specified value to the liquid medicine administration pump.

2. The information processing device according to claim 1, wherein
the liquid medicine administration instruction information includes a plurality of the specified values corresponding to a plurality of the setting items, respectively,
the extraction means extracts each of the specified values from the first data,
the information processing device further includes generation means that generates second data for communicating with the liquid medicine administration pump, by writing each of the extracted specified values to a predetermined communication format, and
the transmission means transmits the second data to the liquid medicine administration pump.

3. The information processing device according to claim 1 or 2, wherein
the liquid medicine administration instruction information includes at least one of a flow rate of a liquid medicine as the specified value, a scheduled volume of the liquid medicine as the specified value, and a scheduled administration time of the liquid medicine as the specified value.

4. The information processing device according to any one of claims 1 to 3, wherein
the scanner reads a pharmaceutical label on which the liquid medicine administration instruction information is written, an injection prescription on which the liquid medicine administration instruction information is written, a display screen of an electronic medical record on which the liquid medicine administration instruction information is written, or a paper-based medical record on which the liquid medicine administration instruction information is written, thereby generating the first data.

5. The information processing device according to claim 2, wherein
the information processing device includes a terminal device and a server device capable of communicating with the terminal device,
the server device includes the extraction means and the generation means, and
the terminal device includes the scanner and the transmission means.

6. The information processing device according to claim 5, wherein
the server device includes a trained model that functions as the extraction means and the generation means, and generates the second data from the first data using the trained model.

7. The information processing device according to any one of claims 1 to 6, wherein
the liquid medicine administration instruction information is provided as a two-dimensional code, and
the scanner includes an application program that reads the two-dimensional code.

8. The information processing device according to any one of claims 1 to 7, wherein
the transmission means transmits the specified value to the liquid medicine administration pump through near field communication.

9. The information processing device according to claim 8, wherein
the liquid medicine administration pump has a non-contact-type integrated circuit card,
the information processing device is a portable device including a reader/writer that functions as at least the transmission means, and
when the information processing device is brought closer to the liquid medicine administration pump, the information processing device transmits the specified value to the liquid medicine administration pump using the reader/writer.

10. The information processing device according to any one of claims 1 to 9, further comprising:
input accepting means that accepts an input to change the extracted specified value; and
change means that changes the specified value to a value based on the input, wherein
the transmission means transmits the changed specified value to the liquid medicine administration pump.

11. A medical system comprising:
the information processing device according to any one of claims 1 to 10; and
the liquid medicine administration pump.

12. An information processing method comprising:
reading, with a sensor, liquid medicine administration instruction information including a specified value of a setting item for a liquid medicine administration pump, thereby generating data;
extracting the specified value from the generated data; and
transmitting the extracted specified value to the liquid medicine administration pump.
